(19) Europäisches Patentamt
European Patent Office
Office européen des brevets

(11) **EP 4 031 009 B1**

(12) **EUROPÄISCHE PATENTSCHRIFT**

(45) Veröffentlichungstag und Bekanntmachung des Hinweises auf die Patenterteilung:
**28.06.2023   Patentblatt 2023/26**

(21) Anmeldenummer: **20775850.9**

(22) Anmeldetag: **19.09.2020**

(51) Internationale Patentklassifikation (IPC):
**A61B 6/03** *(2006.01)*     **A61B 6/00** *(2006.01)*
**H01J 35/24** *(2006.01)*

(52) Gemeinsame Patentklassifikation (CPC):
**A61B 6/4014; A61B 6/032; A61B 6/035; A61B 6/4021; H01J 35/30;** H01J 35/153; H01J 2235/068

(86) Internationale Anmeldenummer:
**PCT/EP2020/076204**

(87) Internationale Veröffentlichungsnummer:
**WO 2021/053203 (25.03.2021 Gazette 2021/12)**

(54) **COMPUTERTOMOGRAPH**

COMPUTER TOMOGRAPH

TOMODENSITOMÈTRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.09.2019   DE 102019125350**

(43) Veröffentlichungstag der Anmeldung:
**27.07.2022   Patentblatt 2022/30**

(73) Patentinhaber: **Esspen GmbH**
**91054 Erlangen (DE)**

(72) Erfinder: **MOHAMMADI, Zahra**
**91052 Erlangen (DE)**

(74) Vertreter: **Meyer, Rudolf**
**Meyer & Dörring**
**Partnerschaft mbB Patentanwälte**
**Nürnberger Straße 49**
**91052 Erlangen (DE)**

(56) Entgegenhaltungen:
DE-A1- 3 426 934     DE-A1-102007 046 278
DE-A1-102010 020 604     DE-A1-102016 013 533

**Beschreibung**

Die Erfindung betrifft einen Computertomographen, dessen Röntgenstrahler-Detektor-Anordnung beim Betrieb des Computertomographen nicht rotiert.

Ein solcher Computertomograph ist beispielsweise aus der WO 2018/086744 A2 bekannt.

Dieser Computertomograph ist insbesondere für die computertomographische Röntgenbildgebung des menschlichen Kopfes geeignet und umfasst eine rotationsfeste Gantry, welche eine Mehrzahl an Röntgenemittern und Röntgendetektoren aufweist, die fest um die geometrische Mittelachse des Computertomographen angeordnet sind. Hierbei sind die Röntgenemitter und die zugehörigen Detektoren in Richtung der Mittelachse gegeneinander versetzt. Die Gantry ist insgesamt in Längsrichtung des Computertomographen, das heißt in Richtung der Mittelachse, verschiebbar.

Aus der DE 10 2007 046 278 A1 ist eine Röntgenröhre mit Transmissionsanode bekannt. Zur Kühlung der Anode kann Wasser oder eine ölige Flüssigkeit verwendet werden. Die Anode ist als Transmissionsanode derart gestaltet, dass genutzte Röntgenstrahlung das Kühlmittel durchtritt. Um die Schwächung der Röntgenstrahlung möglichst gering zu halten, wird die Verwendung eines Kühlmittels empfohlen, welches aus einem Material mit möglichst geringer Kernladungszahl besteht.

Die DE 10 2010 020 604 A1 offenbart einen allgemein als Bildaufnahmevorrichtung bezeichneten Computertomographen mit einer ringförmigen Gantry. In der Gantry rotiert eine Rotoranordnung mit einer Strahlungsquelle sowie mindestens einem Strahlungsdetektor. Die Gantry weist ein zum seitlichen Öffnen der Gantry herausnehmbares Gantrysegment auf und ist an einer deckenseitigen Tragstruktur im Raum bewegbar angeordnet. Die Tragstruktur ist als ein Stativ mit zwei Bewegungsfreiheitsgraden gestaltet. In der Gantry der Bildaufnahmevorrichtung nach der DE 10 2010 020 604 A1 befinden sich wenigstens zwei um einen Winkel versetzt angeordnete Strahlungsquellen, denen jeweils mindestens ein Strahlungsdetektor zugeordnet ist.

Eine weitere computergesteuerte Tomographieeinrichtung ist in der DE 34 26 934 A1 beschrieben. Auch in diesem Fall sind zwei getrennte Strahlungsquellen vorgesehen. Die Vorrichtung nach der DE 34 26 934 A1 soll derart betrieben werden, dass die mindestens zwei getrennten Strahlungsquellen abwechselnd Strahlung emittieren.

Ein in der RU 216 081 C2 beschriebenes röntgentechnisches Gerät weist eine zentrisch in dem Gerät angeordnete Elektronenquelle auf, die Elektronenstrahlen emittiert, die auf eine als Röntgenquelle vorgesehene Anode gerichtet sind, welche das zu untersuchende Objekt ringförmig umgibt.

Ein weiterer Computertomograph ist in der DE 102 37 546 B4 beschrieben. Es handelt sich hierbei um ein Röntgen-Computertomographie-Gerät mit Filter, wobei der Filter auf die richtungsabhängige Intensitätsverteilung eines gefächerten Röntgenstrahls abgestimmt ist. Damit soll dem Heel-Effekt, welcher besonders bei Schräganoden von Röntgenröhren auftritt, begegnet werden.

Der Erfindung liegt die Ausgabe zugrunde, einen gegenüber dem Stand der Technik weiterentwickelten Computertomographen mit feststehender Gantry anzugeben, welcher sich durch eine besonders gute Raumausnutzung bei zugleich günstigen röntgentechnischen Eigenschaften auszeichnet.

Diese Aufgabe wird erfindungsgemäß gelöst durch einen Computertomographen mit den Merkmalen des Anspruchs 1. Der Computertomograph weist in an sich bekannter Grundkonzeption eine Mehrzahl an in festgelegter Winkelstellung, das heißt nicht drehbar, um eine geometrische Mittelachse des Computertomographen angeordnete Röntgenröhren auf. Die Röntgenröhren sind Komponenten von Strahler-Detektor-Elementen, die zusammen einen Strahler-Detektor-Ring bilden, der durch Verlagerung mindestens eines der Strahler-Detektor-Elemente geöffnet werden kann.

Jede Röntgenröhre umfasst mindestens eine zur Emission von Elektronen vorgesehene Kathode, typischerweise mehrere Kathoden, und eine zugehörige Anode, wobei jede Kathode einen Ausrichtungswinkel $\alpha$ aufweist, der durch die mittlere Emissionsrichtung der Elektronen, zu messen gegenüber der geometrischen Mittelachse des Strahler-Detektor-Rings, definiert ist. Die von der Kathode emittierten Elektronen treffen an der Oberfläche der Anode in grundsätzlich bekannter Weise unter Bildung eines Brennflecks auf. Eine mittig am Brennfleck angelegte Tangentialebene weist eine Flächennormale auf, die einen Anodenwinkel $\beta$ mit der Mittelachse des Strahler-Detektor-Rings und damit des gesamten Computertomographen einschließt. Die vom Brennfleck ausgehende Röntgenstrahlung ist in einem mittleren Abstrahlwinkel $\gamma$, zu messen gegenüber einer durch den Brennfleck laufenden, orthogonal die Mittelachse schneidenden Radiallinie, auf einen Röntgendetektor gerichtet, wobei der Abstrahlwinkel $\gamma$ zwischen dem zentralen Strahl des vom Brennfleck ausgehenden Röntgenstrahlenbündels und der genannten Radiallinie zu messen ist. Der Röntgendetektor ist gegenüber der Röntgenröhre in Axialrichtung, bezogen auf die geometrische Mittelachse des Computertomographen, versetzt angeordnet.

Gemäß Anspruch 1 ist zwischen den Winkeln $\alpha$, $\beta$, $\gamma$ folgender Zusammenhang gegeben:

$$2/9 \leq (\alpha + \gamma)/\beta \leq 2$$

Dieser Zusammenhang gilt für sämtliche, jeweils von einem Brennfleck ausgehenden Röntgenstrahlenbündel. Hierbei ist jede als Elektronen-Emitter fungierende Kathode in Zusammenwirkung mit einer in derselben Röntgenröhre befindlichen Elektrodenanordnung zur Erzeugung eines Brennflecks an einer von mindestens drei wählbaren Positionen auf der Anodenanordnung der Röntgenröhre ausgebildet. Die verschiedenen, mit ein und demselben Elektronen-Emitter einstellbaren Positionen des Brennflecks sind hierbei insbesondere in Umfangsrichtung des Strahler-Detektor-Rings nebeneinander angeordnet, das heißt in unterschiedlichen Winkelpositionen um die Mittelachse des Computertomographen verteilt. Insbesondere sind die mit einem einzigen Elektronen-Emitter erzeugbaren Brennfleck äquidistant am Umfang des Strahler-Detektor-Rings angeordnet. Die Winkelabstände zwischen den einzelnen Brennflecken betragen beispielsweise einige Grad oder im Extremfall auch nur ein Bruchteil eines Grades, wobei in jedem Fall einzelne Brennflecke voneinander unterscheidbar sind. Die mittels Ansteuerung der Elektrodenanordnung erfolgende Umschaltung zwischen verschiedenen diskreten Brennfleckpositionen wird auch als Beam Toggling bezeichnet. Dank des Beam Togglings entspricht die Gesamtzahl möglicher Brennfleckpositionen einem Mehrfachen der Anzahl an Elektronen-Emittern des Computertomographen. Das Beam Toggling kann in gleicher Weise sowohl bei dem mindestens einen zum Zweck der Öffnung des Strahler-Detektor-Rings verlagerbaren Strahler-Detektor-Element als auch bei den übrigen Strahler-Detektor-Elementen erfolgen.

Die Erfindung geht von folgenden Überlegungen aus:

Die von der Kathode einer in einen Computertomographen eingebauten Röntgenröhre emittierten Elektronen können prinzipiell mit einer Hauptausbreitungsrichtung parallel zur Mittelachse des Computertomographen ausgestrahlt werden, wobei die Elektronenstrahlung beeinflussende Mittel, insbesondere in Form von Fokussierungselektroden, vorgesehen sein können. Durch das Auftreffen der Elektronen auf die Anode der Röntgenröhre bildet sich ein Brennfleck auf der Anodenoberfläche aus. Typischerweise sind Röntgenröhren derart gestaltet, dass die Elektronen in einem Winkel ungleich 90° auf die Anodenoberfläche auftreffen. Beispielsweise ergibt sich bei einer im Wesentlichen linienförmigen Elektronenquelle eine ebenfalls längliche Form des Brennflecks.

Die längliche Form des Brennflecks kann optisch verkürzt werden, indem durch Blenden ein Röntgenstrahlenbündel geformt wird, das schräg von der Oberfläche der Anode abstrahlt. Hierbei ist jedoch zu berücksichtigen, dass ein nahe an der Oberfläche der Anode liegender Ausschnitt aus dem Röntgenstrahlenbündel durch den Heel-Effekt geschwächt wird. Dieser Effekt tritt umso mehr auf, je mehr die emittierte Röntgenstrahlung von einer zur Mittelachse des Computertomographen normalen Ebene, in welcher der Brennfleck liegt, weg gestrahlt werden soll. Eine Abstrahlung aus der genannten Ebene heraus ist jedoch notwendig, wenn Röntgenquellen und zugehörige Detektoren nicht in einer gemeinsamen Ebene liegen sollen. Um den Heel-Effekt abzumildern, ist es möglich, die Röntgenröhre als Ganzes gegenüber der Mittelebene des Computertomographen schräg zu stellen, das heißt einen Ausrichtungswinkel $\alpha$ größer Null zu wählen. Dies vergrößert allerdings den benötigten Bauraum in radialer Richtung, bezogen auf die Mittelachse des Computertomographen.

Dem erläuterten Zielkonflikt wird gemäß der Erfindung dadurch Rechnung getragen, dass der der Quotient, welcher aus der Summe aus dem Ausrichtungswinkel $\alpha$ der Röntgenröhre und dem Abstrahlwinkel $\gamma$ als Zähler und aus dem Anodenwinkel $\beta$ als Nenner gebildet ist, mindestens zwei Neuntel und höchstens zwei beträgt. Insbesondere sind Ausgestaltungen realisierbar, in welchen der genannte Quotient mindestens zwei Fünftel und höchstens acht Fünftel, beispielsweise mindestens 1 und maximal 1,6, beträgt. In allen Fällen ist der Quotient dimensionslos.

Die Möglichkeit, den Strahler-Detektor-Ring, welcher nicht notwendigerweise eine kreisrunde Grundform hat, zu öffnen, bietet sowohl bei der Vorbereitung einer röntgentechnischen Untersuchungen als auch bei Tätigkeiten, insbesondere medizinischen Eingriffen, die nach einer ersten und vor einer weiteren röntgentechnischen, das heißt im vorliegenden Fall computertomographischen Untersuchung durchgeführt werden, praktische Vorteile. Insbesondere kann die Lage eines Patienten auf einer Patientenliege unverändert bleiben, auch wenn die Generierung computertomographischer Aufnahmen unterbrochen wird, um Eingriffe vorzunehmen, bei welchen die geschlossene Strahler-Detektor-Anordnung im Weg wäre. In einem solchen Fall kann der bewegliche Teil des Strahler-Detektor-Rings aus dem Arbeitsbereich entfernt werden, wobei der restliche, starre Teil des Strahler-Detektor-Rings in seiner ursprünglichen, für die computertomographische Bildgebung erforderlichen Position verbleibt. Bei einer erneuten computertomographischen Untersuchung ist somit, soweit keine Änderung des zu untersuchenden Volumens gewünscht ist, keine erneute Einstellung der Position des Patienten oder des Strahler-Detektor-Rings erforderlich.

Der Strahler-Detektor-Ring ist unabhängig von seiner Form vorzugsweise aus einer ungeraden Anzahl an Strahler-Detektor-Elementen aufgebaut. Mit der ungeraden Anzahl an Strahler-Detektor-Elementen kann dafür gesorgt werden, dass keine Stoßstelle zwischen zwei Strahler-Detektor-Elementen einer weiteren derartigen Stoßstelle exakt diametral gegenüberliegt. Befindet sich ein Brennfleck im Randbereich eines Strahler-Detektor-Elements, das heißt nahe einer Stoßstelle zwischen zwei Segmenten des Strahler-Detektor-Rings, so trifft von diesem Brennfleck ausgehende Röntgenstrahlung auf zwei in Umfangsrichtung nebeneinander angeordnete Röntgendetektoren, bei welchen es sich beispielsweise um photonenzählende Detektoren handelt. Ebenso kommt die Verwendung von Zeilendetektoren in Betracht. In diesem Zusammenhang wird auf die WO 2019/057339 A1 hingewiesen.

Unabhängig von der - beispielsweise geraden gekrümmten - Form der einzelnen Strahler-Detektor-Ele-

mente kann der Ausrichtungswinkel $\alpha$ der Kathode, welcher sich durch die Winkellage der gesamten Röntgenröhre einstellen lässt, größer als Null, insbesondere größer als 5°, sein. Dies bedeutet, dass der von der Kathode ausgehende Elektronenstrahl zumindest geringfügig radial nach außen, also von der Mittelachse des Computertomographen weg, gerichtet ist. Vorzugsweise beträgt der Ausrichtungswinkel nicht mehr als 30°.

Der Anodenwinkel $\beta$ beträgt beispielsweise mindestens 10° und maximal 60°. Im Fall eines Anodenwinkels $\beta$ von 10° und eines beliebigen, bevorzugt positiven Ausrichtungswinkels $\alpha$, schließt eine Flächennormale einer im Brennfleck an die Anode gelegten Ebene, das heißt Tangentialebene, mit der Mittelachse des Computertomographen einen Winkel von 10° ein. Der mittlere Abstrahlwinkel $\gamma$, welcher die Abstrahlung von Röntgenstrahlung vom Brennfleck betrifft, beträgt beispielsweise mindestens 5° und maximal 30°.

Wird zum Beispiel ein Ausrichtungswinkel $\alpha$ von 12°, ein Anodenwinkel $\beta$ von 20° und ein Abstrahlwinkel $\gamma$ von 10° gewählt, so beträgt der genannte Quotient 1,1. Ebenso sind beispielsweise Bauformen realisierbar, in welchen der Ausrichtungswinkel $\alpha$ = 30°, der Anodenwinkel $\beta$ = 30° und der Abstrahlwinkel $\gamma$ = 18° beträgt. In diesem Fall ergibt sich ein Quotient $(\alpha + \gamma) / \beta$ von 1,6. Ein Quotient, der weniger als 1, nämlich zwei Fünftel, beträgt, ist zum Beispiel bei Bauformen mit einem Ausrichtungswinkel $\alpha$ von 0°, einem Anodenwinkel $\beta$ von 30° und einem Abstrahlwinkel $\gamma$ von 12° gegeben. Derselbe Wert von $(\alpha + \gamma) / \beta$ ergibt sich auch bei Bauformen mit einem Ausrichtungswinkel $\alpha$ von 6°, einem Anodenwinkel $\beta$ von 45° und einem Abstrahlwinkel $\gamma$ von 12°.

Gemäß einer ersten möglichen Bauform des Computertomographen beschreibt der durch die Strahler-Detektor-Elemente gebildete Strahler-Detektor-Ring eine segmentierte Kreisform, wobei mindestens ein als Segment ausgebildetes Strahler-Detektor-Element gegenüber den übrigen Strahler-Detektor-Elementen verlagerbar, insbesondere verschwenkbar, ist. Sind mehrere verschwenkbare Segmente vorhanden, so können diese beispielsweise untereinander starr verbunden und als Ganzes aus dem restlichen Strahler-Detektor-Ring ausschwenkbar sein. Alternativ ist es möglich, mehrere verschwenkbare Segmente in der Art einer Flügeltür am restlichen Strahler-Detektor-Ring zu lagern.

Gemäß einer weiteren möglichen Bauform beschreibt der durch die Strahler-Detektor-Elemente gebildete Strahler-Detektor-Ring eine Polygonform, insbesondere Rechteckform, wobei mehrere starr miteinander verbundene Strahler-Detektor-Elemente vorhanden sein können, welche gegenüber dem restlichen Strahler-Detektor-Ring verschwenkbar sind. In jeglicher Bauform kann im Fall einer einheitlichen Gestaltung sämtlicher Strahler-Detektor-Elemente die Ansteuerung dieser Elemente auf eine einheitliche Art erfolgen, unabhängig davon, ob es sich bei einem einzelnen Strahler-Detektor-Element um ein zum Öffnen vorgesehenes Element handelt.

Möglichkeiten der Ansteuerung, welche auch im vorliegenden Fall genutzt werden können, sind zum Beispiel in der WO 2019/042587 A2 beschrieben. Bei der Herstellung der zur Emission von Elektronen ausgebildeten Kathoden kann zum Beispiel auf jegliche Lösungen zurückgegriffen werden, die in den Dokumenten WO 2018/086737 A1 und WO 2018/141485 A1 erwähnt sind. Insgesamt können die Röntgenröhren des Computertomographen dazu ausgebildet sein, eine Folge von Röntgenpulsen zu erzeugen, welche sich hinsichtlich verschiedenster Parameter, unter anderem Dauer und Röntgendosis der einzelnen Pulse sowie Frequenz der Röntgenstrahlung, voneinander unterscheiden.

Bei der Anode handelt es sich vorzugsweise um eine feststehende Anode, das heißt eine nicht innerhalb des Anodengehäuses rotierende Anode. Die Anode kann entweder als flüssigkeitsgekühlte Anode, das heißt als Anode, welche von einem Kühlmittel durchströmt ist, oder als Anode ohne Kühlkanal ausgebildet sein. Im letztgenannten Fall wird kurz von einer ungekühlten Anode gesprochen.

Beispielsweise können Anoden zum Einsatz kommen, wie sie in der DE 10 2017 008 810 A1 beschrieben sind. Was die Gestaltung der Kathoden, welche als Elektronen-Emitter des Computertomographen verwendbar sind, betrifft, können zum Beispiel Möglichkeiten gewählt werden, die in der WO 2019/057338 A1, welche die Priorität der genannten Patentanmeldung der DE 10 2017 008 810 A1 in Anspruch nimmt, gewählt werden.

Die Kathode ist in bevorzugter Ausgestaltung zur Feldemission von Elektronen ausgebildet. Insbesondere umfasst die Kathode Nanostäbchen, beispielsweise Kohlenstoffnanoröhren. Beispiele möglicher Materialien der Kathode sind in der WO 2018/086737 A1 aufgeführt.

Gemäß einer möglichen Weiterbildung umfasst jedes Strahler-Detektor-Element mindestens einen Elektronen-Emitter ersten Typs sowie mindestens einen Elektronen-Emitter zweiten Typs, wobei sich die unterschiedlichen Emitter-Typen innerhalb eines Strahler-Detektor-Elements hinsichtlich ihrer Materialien und/oder Geometrie voneinander unterscheiden.

Je nach Dimensionierung der Strahler-Detektor-Anordnung kann der Computertomograph beispielsweise zur Untersuchung des menschlichen Kopfes, für Untersuchungen der Brust oder für Ganzkörperuntersuchungen geeignet sein.

Die Detektoren des Computertomographen sind beispielsweise als Halbleiterdetektoren aufgebaut. Hinsichtlich einer besonders hohen Empfindlichkeit sind photonenzählende Detektoren als Komponenten des Computertomographen von Vorteil. In diesem Zusammenhang wird beispielhaft auf das Dokument DE 10 2014 215 548 A1 hingewiesen.

Nachfolgend werden mehrere Ausführungsbeispiel der Erfindung anhand einer Zeichnung näher erläutert. Hierin zeigt:

Fig. 1      einen Computertomographen in schematisierter Darstellung,

Fig. 2     den Computertomographen nach Fig. 1 in vereinfachter stirnseitiger Ansicht mit geöffnetem Strahler-Detektor-Ring,

Fig. 3     die Anordnung nach Fig. 2 mit geschlossenem Strahler-Detektor-Ring,

Fig. 4 und 5     in Ansichten analog Fig. 2 und 3 eine modifizierte Bauform eines Computertomographen,

Fig. 6 und 7     in weiteren Ansichten analog Fig. 2 und 3 eine Bauform eines Computertomographen mit rechteckigem Strahler-Detektor-Ring,

Fig. 8 und 9     in schematisierten Darstellungen Einzelheiten eines Computertomographen mit einem kreisrunden, segmentierten Strahler-Detektor-Ring.

Die folgenden Erläuterungen beziehen sich, soweit nicht anders angegeben, auf sämtliche Ausführungsbeispiele. Einander entsprechende oder prinzipiell gleichwirkende Teile sind in allen Figuren mit den gleichen Bezugszeichen gekennzeichnet.

Ein insgesamt mit 1 bezeichneter Computertomograph ist speziell für die Untersuchung des menschlichen Kopfes konzipiert, das heißt als Kopf-CT ausgebildet. Hinsichtlich des prinzipiellen Aufbaus des Computertomographen 1 wird auf das eingangs zitierte Dokument WO 2018/086744 A2 verwiesen.

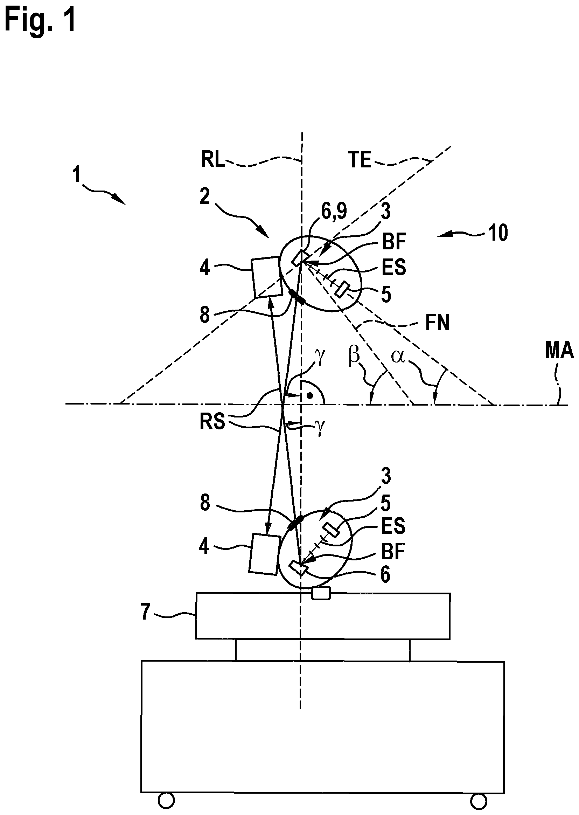

Der Computertomograph 1 ist in Fig. 1 in einer grob schematisierten Schnittdarstellung gezeigt, wobei die mit MA bezeichnete Mittelachse des Computertomographen 1 in der Bildebene liegt. Durch die Mittelachse MA ist die z-Richtung eines kartesischen Koordinatensystems gegeben. Die x- und y-Achse des Koordinatensystems spannen eine zur Bildebene orthogonale Ebene auf. Eine insgesamt mit 2 bezeichnete Gantry des Computertomographen hat die Grundform eines Ringes, der in der x-y-Ebene liegt. Dies bedeutet, dass die Ebene, in welcher die Gantry 2 liegt, normal zur Mittelachse MA ausgerichtet ist.

In den Ausführungsbeispielen haben die Röntgenröhren 3 im Querschnitt, wie aus Fig. 1 hervorgeht, ein elliptische, nicht kreisrunde Form. Alternativ könnten beispielsweise auch Röntgenröhren zum Einsatz kommen, deren Querschnittsform kreisrund oder polygonförmig, zum Beispiel quadratisch, hexagonal oder achteckig, ist.

Die Gantry 2 umfasst eine Mehrzahl an Röntgenröhren 3, welche beim Betrieb des Computertomographen 1 in starrer Winkelanordnung um die Mittelachse MA verteilt sind. Zugehörige Röntgendetektoren 4, nämlich Halbleiterdetektoren, sind ebenso in einem Ringraum um die Mittelachse MA angeordnet. Die gesamte Anordnung aus Röntgenröhren 3 und Röntgendetektoren 4 ist nicht drehbar, sondern lediglich in z-Richtung, das heißt in Längsrichtung der Mittelachse MA, verschiebbar. Zu diesem Zweck ist die Gantry 2 auf einem fahrbaren Gestell 7 montiert. Die Ebene, in welcher die Röntgenröhren 3 liegen, ist gegenüber der Ebene, in welcher die Röntgendetektoren 4 liegen, in Axialrichtung, das heißt z-Richtung, verschoben.

Jede Röntgenröhre 3 weist eine Mehrzahl an Kathoden 5 als Elektronenemitter auf. Eine zugehörige Anode ist mit 6 bezeichnet und einer Anodenanordnung 9 der entsprechenden Röntgenröhre 9 zuzurechnen. Vorrichtungen zur Beeinflussung des mit ES bezeichneten Elektronenstrahls, insbesondere Fokussierungselektroden, sind der Übersichtlichkeit halber in Fig. 1 nicht dargestellt. Der Elektronenstrahl ES, bezogen auf die Strahlmitte, schließt mit der Mittelachse MA einen Winkel ein, der als Ausrichtungswinkel $\alpha$ der Kathode 5 bezeichnet wird. In den Ausführungsbeispielen ist der Ausrichtungswinkel $\alpha$ größer als Null. Dies bedeutet, dass die von der Kathode 5 emittierten Elektronen eine Bewegungskomponente radial nach außen, bezogen auf die Mittelachse MA, haben.

Die von der Kathode 5 emittierten Elektronen treffen an einem allgemein mit BF bezeichneten Brennfleck auf der Anode 6 auf. In den Ausführungsbeispielen ist die Anode 6 nicht drehbar, das heißt als statische Anode, ausgebildet. Alternativ könnten Drehtelleranoden zum Einsatz kommen. In solchen Fällen wäre der Elektronenstrahl ES vorzugsweise parallel zur Rotationsachse der Drehtelleranode ausgerichtet. Der Ausrichtungswinkel $\alpha$ entspräche somit dem Schrägstellungswinkel der Rotationsachse der Drehtelleranode gegenüber der Mittelachse MA.

Eine Flächennormale FN einer Tangentialebene TE, die an die Anode 6 gelegt ist und in der der Brennfleck BF liegt, schließt mit der Mittelachse MA einen Anodenwinkel $\beta$ ein. Der Anodenwinkel $\beta$ beträgt in allen in den Figuren veranschaulichten Ausführungsbeispielen mindestens 10° und nicht mehr als 45°.

Vom Brennfleck BF geht Röntgenstrahlung RS aus, wobei in Fig. 1 lediglich der zentrale Strahl eines Röntgenstrahlenbündels skizziert ist. Die Ausrichtung der für die Bildgebung zu verwendenden Röntgenstrahlung RS wird in an sich bekannter Weise durch Blenden bestimmt. Für den Austritt von Röntgenstrahlung RS weist die Röntgenröhre 3 ein Röntgenfenster 8 auf.

Die Röntgenstrahlung RS tritt in einem mittleren Abstrahlwinkel $\gamma$, zu messen gegenüber einer Radiallinie RL, aus der Röntgenröhre 3 aus. Die Radiallinie RL ist orthogonal zur Mittelachse MA ausgerichtet und schneidet die Mittelachse MA sowie die Mitte des Brennflecks BF. Ein Abstrahlwinkel $\gamma$ von Null Grad würde also bedeuten, dass die Röntgenstrahlung RS genau in Radialrichtung, das heißt orthogonal zur Mittelachse MA, ausgerichtet ist. Aufgrund des Versatzes der Röntgendetektoren 4 gegenüber den Röntgenröhren 3 in Axialrichtung, bezogen auf die Mittelachse MA, muss der Abstrahlwinkel γ größer als Null sein. In den Ausführungsbeispielen beträgt der Abstrahlwinkel γ mindestens 5°, jedoch nicht mehr als 30°.

Die Summe aus dem Abstrahlwinkel γ und dem Ausrichtungswinkel α beträgt mindestens ein Zwölftel und maximal das Doppelte des Anodenwinkels β.

Die Kathoden 5 sind in den Ausführungsbeispielen als Feldemissionskathoden ausgebildet. Hierbei ist in den einzelnen Röntgenröhren 3 jeweils eine Mehrzahl an Kathoden 5 einer gemeinsamen Anode 6 zugeordnet. Dabei können die einzelnen Röntgenröhren 3 jeweils mehrere Kathoden 5, 25 einheitlicher oder unterschiedlicher Gestaltung als Elektronenemitter aufweisen.

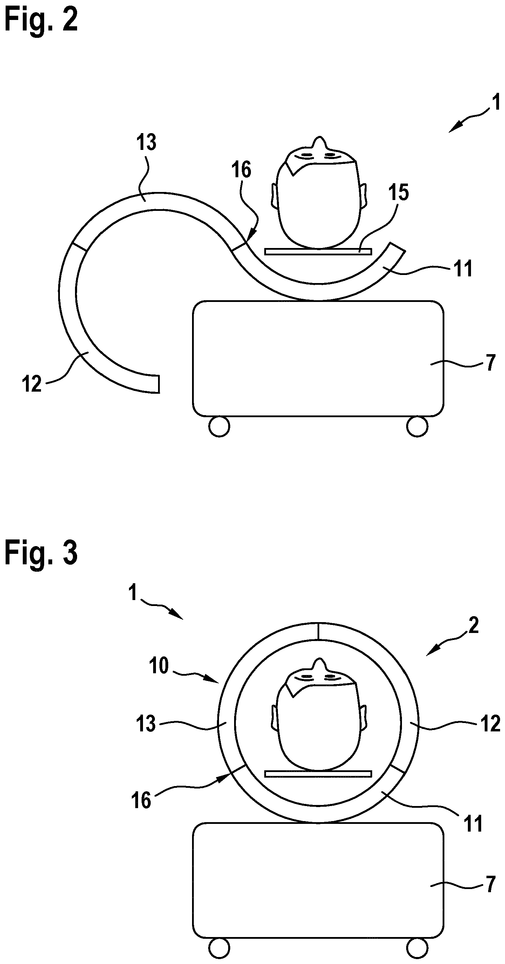

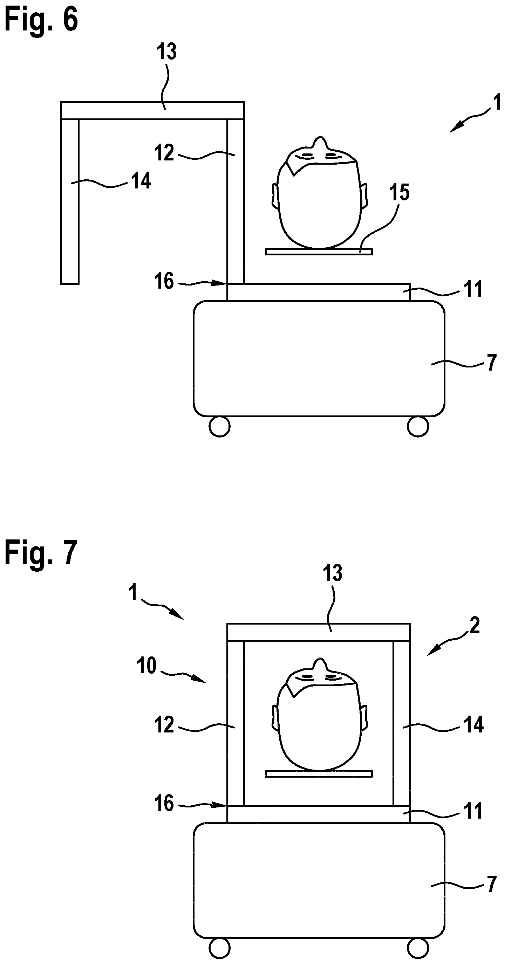

Jede Röntgenröhre 3 ist Teil eines Strahler-Detektor-Elementes 11, 12, 13, 14, dem jeweils auch ein Röntgendetektor 4 zuzurechnen ist. Die Gesamtheit der Strahler-Detektor-Elemente 11, 12, 13, 14 bildet einen nicht rotierenden Strahler-Detektor-Ring 10, welcher die Gantry 2 darstellt und in den Ausführungsbeispielen nach den Figuren 1 bis 5 sowie 8 und 9 eine kreisrunde Form hat. Im Ausführungsbeispiel nach den Figuren 6 und 7 hat der Strahler-Detektor-Ring 10 dagegen eine quadratische Grundform, was ohne Einfluss auf dessen wesentliche Grundfunktionen, sowohl in röntgentechnischer Hinsicht als auch unter dem Aspekt der Handhabung, ist. Ein die Gantry 2 umschließendes Gehäuse ist nicht vorgesehen. In allen Fällen ist der Begriff "feststehend", der die Anordnung des Strahler-Detektor-Rings 10 beschreibt, dahingehend zu verstehen ist, dass bei der Gewinnung röntgentechnischer Aufnahmen keine Rotation einer Strahler-Detektor-Einheit um die Mittelachse MA der Gantry 2 gegeben ist. Vielmehr sind mit Hilfe um den gesamten Umfang der Gantry 2 verteilter Röntgenröhren 3 und zugehöriger Röntgendetektoren 4 fächerförmige Bündel an Röntgenstrahlung RS erzeugbar, welche jeweils von einem Brennfleck BF auf einer Anode 6 der Röntgenröhre 3 ausgehen.

In den Ausführungsbeispielen nach den Figuren 1 bis 5 ist der Strahler-Detektor-Ring 10, der aus insgesamt drei Strahler-Detektor-Elementen 11, 12, 13 aufgebaut, wobei das Strahler-Detektor-Element 11 auf dem Gestell 7 montiert ist und damit als feststehende Strahler-Detektor-Element gilt. Dagegen ermöglichen die Strahler-Detektor-Elemente 12, 13 ein Öffnen des Strahler-Detektor-Rings 10, um diesen zum Beispiel von der Seite über eine Patientenliege 15 schieben zu können.

Zum Öffnen des Strahler-Detektor-Rings 10 ist im Fall der Figuren 1 bis 3 ein einziges Scharnier 16 vorgesehen, welches zwischen den Strahler-Detektor-Elementen, das heißt Segmenten 11, 13 angeordnet ist. Die Segmente 12, 13, welche sich insgesamt über ca. 240° am Umfang des Strahler-Detektor-Rings 10 erstrecken, sind in diesem Fall starr miteinander verbunden und werden beim Öffnen und Schließen des Rings 10 gemeinsam verschwenkt.

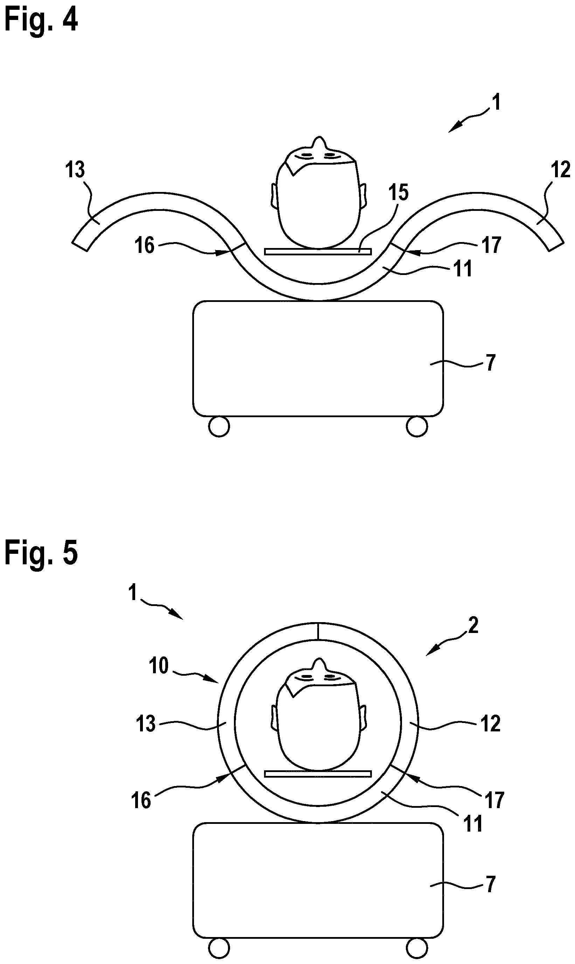

Das Ausführungsbeispiel nach den Figuren 4 und 5 unterscheidet sich von der Bauform nach den Figuren 1 bis 3 dadurch, dass zwei Scharniere 16, 17 vorhanden sind, an denen das Segment 13 beziehungsweise das Segment 12 schwenkbar angelenkt ist. Somit sind die Strahler-Detektor-Elemente 12, 13 in der Art einer Flügeltür zu öffnen.

Das Ausführungsbeispiel nach den Figuren 4 und 5 weist insofern Gemeinsamkeiten mit der Bauform nach den Figuren 1 bis 3 auf, als mehrere Strahler-Detektor-Elemente 12, 13, 14, welche in diesem Fall zusammen eine U-Form beschreiben, gemeinsam verschwenkbar sind. Durch die flache Form sämtlicher Segmente, auch des feststehenden Segmentes 11, ist der Untersuchungsbereich bei geöffnetem Strahler-Detektor-Ring 10 besonders gut zugänglich.

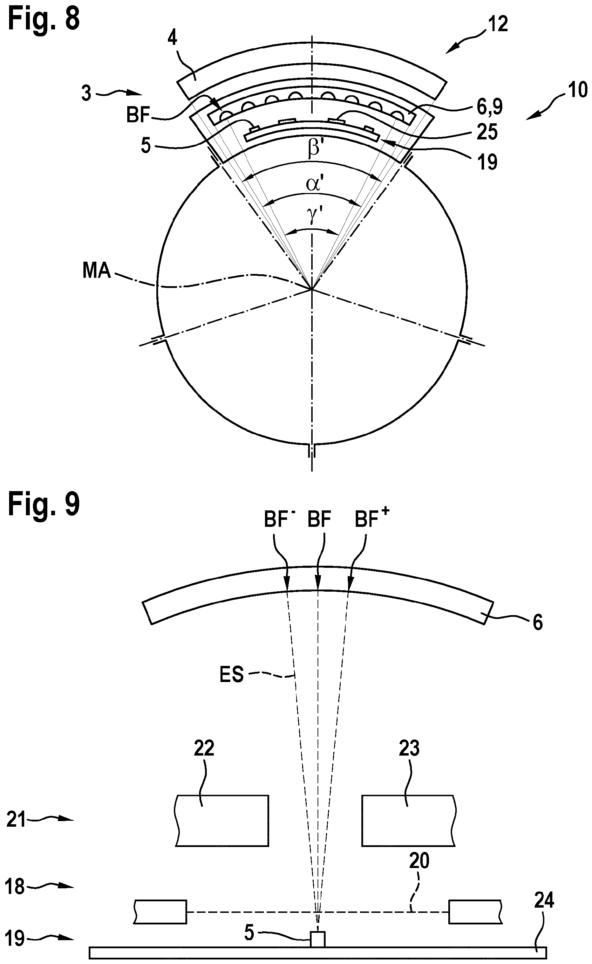

Die Figuren 8 und 9 veranschaulichen Einzelheiten der Röntgenröhren 3 und weiterer röntgentechnischer Komponenten, welche für sämtliche erläuterten Ausführungsbeispiele zutreffend sind. Strahler-Anordnungen der Röntgenröhren 3 sind mit 18 bezeichnet.

In jeder Röntgenröhre 3 befindet sich eine Emitter-Baugruppe 19 zur Erzeugung von Elektronenstrahlen ES, die auf die Anodenanordnung 9 treffen und damit den Brennfleck BF erzeugen. Der Brennfleck BF hat nicht notwendigerweise eine annähernd punktförmige Gestalt. Vielmehr können in prinzipiell bekannter Weise zum Beispiel auch langgestreckte Brennflecke BF erzeugt werden, wobei die Lage des Brennflecks BF in jedem Fall als Lage dessen Mittelpunkts zu verstehen ist.

Gemäß Fig. 8 umfasst die Emitter-Baugruppe 19 unterschiedliche Kathoden 5, 25, um Röntgenstrahlung unterschiedlicher Dosis und/oder Wellenlänge zu erzeugen. In jedem Fall werden Elektronen mit Hilfe eines Extraktionsgitters 20 aus der Kathode 5, 25 extrahiert, wobei der Elektronenstrahl ES mit Hilfe einer Elektrodenanordnung 21, die mehrere Elektroden 22, 23 umfasst, in definierter Weise auslenkbar ist. Eine Mehrzahl an Kathoden 5, 25 ist gemeinsam auf einer Platine 24 angeordnet.

Die gesamte mit der Emitter-Baugruppe 19 einer Röntgenröhre 3 zusammenwirkende Anodenanordnung 9 erstreckt sich am Umfang des Strahler-Detektor-Rings 10 über einen Winkel α', der sich aus der Anzahl der Strahler-Detektor-Elemente 11, 12, 13, 14, das heißt Segmente des Strahler-Detektor-Rings 10, ergibt, wobei in der in Fig. 8 skizzierten Anordnung fünf Segmente gleich großer Ausdehnung, das heißt 120°-Segmente, gegeben sind.

Der Winkel α', der die Erstreckung der Anodenanordnung 9 in Umfangsrichtung des Strahler-Detektor-Rings 10 angibt, beträgt im Fall von Fig. 8 etwas weniger als 120° beträgt. Noch deutlich näher an 72° liegt ein Winkel β', der die Erstreckung des Röntgendetektors 4 am Umfang des Strahler-Detektor-Rings angibt. Anders ausgedrückt: Am Umfang des Strahler-Detektor-Rings 10 zwischen den einzelnen Röntgendetektoren 4 gebildete Lücken sind wesentlich enger als die zwischen den Strahler-Anordnungen 18 gebildeten Lücken. Eine Vielzahl möglicher Brennfleckpositionen erstreckt sich innerhalb der Röntgenröhre 3 über einen Winkel $\gamma'$, der geringer als der Winkel $\alpha'$ ist.

Die Elektrodenanordnung 21 ist dazu ausgebildet, den Elektronenstrahl ES wahlweise auf den Brennfleck BF oder einen im Vergleich hierzu in Umfangsrichtung des Strahler-Detektor-Rings 10 versetzten Brennfleck BF$^+$, BF$^-$ zu richten. Bezogen auf die Anordnung nach den Figuren 8 und 9 ist der Brennfleck BF$^+$ gegenüber dem Brennfleck BF im Uhrzeigersinn und der Brennfleck BF$^-$ im Gegenuhrzeigersinn ausgelenkt. Die Auslenkungen des Elektronenstrahls ES, welche einen Versatz des Brennflecks BF bedeuten, werden auch als Beam Toggling bezeichnet und ermöglichen eine besonders eng gestaffelte Platzierung von Brennflecken BF$^-$, BF, BF$^+$ am Umfang des Strahler-Detektor-Rings 10. Hierbei ist eine Gesamtzahl von mehreren hundert Brennfleckpositionen, was einem Mehrfachen der Anzahl der Elektronenemitter 5, 25 entspricht, erzielbar, womit ein massesparender Aufbau des Computertomographen 1 bei zugleich hoher Qualität der Bildgebung begünstigt wird.

### Bezugszeichenliste

| 1 | Computertomograph |
|---|---|
| 2 | Gantry |
| 3 | Röntgenröhre |
| 4 | Röntgendetektor |
| 5 | Kathode, Elektronenemitter |
| 6 | Anode |
| 7 | Gestell |
| 8 | Röntgenfenster |
| 9 | Anodenanordnung |
| 10 | Strahler-Detektor-Ring |
| 11 | Strahler-Detektor-Element |
| 12 | Strahler-Detektor-Element |
| 13 | Strahler-Detektor-Element |
| 14 | Strahler-Detektor-Element |
| 15 | Patientenliege |
| 16 | Scharnier |
| 17 | Scharnier |
| 18 | Strahler-Anordnung |
| 19 | Emitter-Baugruppe |
| 20 | Extraktionsgitter |
| 21 | Elektrodenanordnung |
| 22 | Elektrode |
| 23 | Elektrode |
| 24 | Platine |
| 25 | Kathode zweiter Art, Elektronenemitter |

| $\alpha$ | Ausrichtungswinkel |
|---|---|
| $\beta$ | Anodenwinkel |
| $\gamma$ | Abstrahlwinkel |
| $\alpha'$ | Winkel, über welchen sich die Anodenanordnung |

eines Strahler-Detektor Elements erstreckt

| $\beta'$ | Winkel, über welchen sich der Detektor eines Strahler-Detektor Elements erstreckt |
|---|---|
| $\gamma'$ | Winkelbereich, in dem die möglichen Brennflecke einer Anodenanordnung liegen |

| BF | Brennfleck (allgemein) |
|---|---|
| BF$^+$, BF$^-$ | Brennfleck, erzeugt mittels eines Elektronenemitters (in mittlerer Position sowie in zwei in Umfangsrichtung des Strahler-Detektor-Rings versetzten Positionen) |
| ES | Elektronenstrahl |
| FN | Flächennormale |
| MA | Mittelachse |
| RL | Radiallinie |
| RS | Röntgenstrahlung |
| TE | Tangentialebene |

### Patentansprüche

1. Computertomograph, mit einer Mehrzahl an in festgelegter Winkelstellung um eine geometrische Mittelachse (MA) angeordneten Röntgenröhren (3), welche Komponenten von Strahler-Detektor-Elementen (11, 12, 13, 14) sind, die zusammen einen Strahler-Detektor-Ring (10) bilden, der durch Verlagerung mindestens eines der Strahler-Detektor-Elemente (11, 12, 13, 14) zu öffnen ist, wobei jede Röntgenröhre (3) mindestens eine zur Emission von Elektronen vorgesehene Kathode (5, 25) und eine zugehörige Anodenanordnung (9) mit mindestens einer Anode (6) umfasst, wobei jede Kathode (5, 25), was die Emissionsrichtung der Elektronen betrifft, einen Ausrichtungswinkel ($\alpha$) gegenüber der geometrische Mittelachse (MA) aufweist, und wobei eine am Brennfleck (BF$^-$, BF, BF$^+$) der Anode (6) angelegte Tangentialebene (TE) eine Flächennormale (FN) aufweist, die einen Anodenwinkel ($\beta$) mit der Mittelachse (MA) einschließt, und wobei die vom Brennfleck (BF$^-$, BF, BF$^+$) ausgehende Röntgenstrahlung (RS) in einem mittleren Abstrahlwinkel ($\gamma$), zu messen gegenüber einer durch den Brennfleck (BF) laufenden Radiallinie (RL), auf einen Röntgendetektor (4) gerichtet ist, der gegenüber der Röntgenröhre (3) in Axialrichtung, bezogen auf die geometrische Mittelachse (MA), versetzt angeordnet ist, wobei der Quotient, welcher aus der Summe aus dem Ausrichtungswinkel ($\alpha$) und dem Abstrahlwinkel ($\gamma$) und aus dem Anodenwinkel ($\beta$) gebildet ist, mindestens zwei Neuntel und höchstens zwei beträgt, und wobei jede Kathode (5, 25) in Zusammenwirkung mit einer Elektrodenanordnung (21) der Röntgenröhre (3) zur Erzeugung eines Brennflecks (BF$^-$, BF, BF$^+$) an einer von mindestens drei wählbaren Positionen auf der Anodenanordnung (9) ausgebildet ist.

2. Computertomograph nach Anspruch 1, **dadurch ge-**

**kennzeichnet, dass** der Ausrichtungswinkel ($\alpha$) größer als Null ist und maximal 30° beträgt.

3. Computertomograph nach Anspruch 1 oder 2, **dadurch gekennzeichnet, dass** der Anodenwinkel ($\beta$) mindestens 10° und maximal 60° beträgt.

4. Computertomograph nach einem der Ansprüche 1 bis 3, **dadurch gekennzeichnet, dass** der mittlere Abstrahlwinkel ($\gamma$) mindestens 5° und maximal 30° beträgt.

5. Computertomograph nach einem der Ansprüche 1 bis 4, **dadurch gekennzeichnet, dass** die Anode (6) als feststehende Anode ausgebildet ist.

6. Computertomograph nach Anspruch 5, **dadurch gekennzeichnet, dass** die Anode (6) als ungekühlte Anode ausgebildet ist.

7. Computertomograph nach Anspruch 5, **dadurch gekennzeichnet, dass** die Anode (6) als flüssigkeitsgekühlte Anode ausgebildet ist

8. Computertomograph nach einem der Ansprüche 1 bis 7, **dadurch gekennzeichnet, dass** der Quotient, welcher aus der Summe aus dem Ausrichtungswinkel ($\alpha$) und dem Abstrahlwinkel ($\gamma$) als Zähler und aus dem Anodenwinkel ($\beta$) als Nenner gebildet ist, mindestens 2/5 und höchstens 8/5 beträgt.

9. Computertomograph nach einem der Ansprüche 1 bis 8, **dadurch gekennzeichnet, dass** die Kathode (5) zur Feldemission von Elektronen ausgebildet ist und Nanostäbchen, insbesondere Kohlenstoffnanoröhren, umfasst.

10. Computertomograph nach Anspruch 9, **dadurch gekennzeichnet, dass** jedes Strahler-Detektor-Element (11, 12, 13, 14) mindestens einen Emitter (5) ersten Typs sowie mindestens einen Emitter (25) zweiten Typs aufweist, wobei sich die unterschiedlichen Emitter-Typen (5, 25) innerhalb eines Strahler-Detektor-Elements (11, 12, 13, 14) hinsichtlich ihrer Materialien und/oder Geometrie voneinander unterscheiden.

11. Computertomograph nach Anspruch 9 oder 10, **dadurch gekennzeichnet, dass** die Strahler-Detektor-Elemente (11, 12, 13, 14) zur Umschaltung zwischen verschiedenen Röntgenfrequenzen und/oder Röntgendosen ausgebildet sind, wobei jeder Brennfleck (BF⁻, BF, BF⁺) gleichermaßen als Quelle sämtlicher einstellbarer Röntgenfrequenzen und Röntgendosen wählbar ist.

12. Computertomograph nach einem der Ansprüche 1 bis 11, **dadurch gekennzeichnet, dass** der durch die Strahler-Detektor-Elemente (11, 12, 13, 14) gebildete Strahler-Detektor-Ring (10) eine segmentierte Kreisform beschreibt, wobei mindestens ein als Segment ausgebildetes Strahler-Detektor-Element (11, 12, 13, 14) gegenüber den übrigen Strahler-Detektor-Elementen (11, 12, 13, 14) verlagerbar, insbesondere verschwenkbar, ist.

13. Computertomograph nach Anspruch 12, **dadurch gekennzeichnet, dass** jedes zu öffnende Strahler-Detektor-Element (11, 12, 13, 14) einzeln verschwenkbar ist.

14. Computertomograph nach einem der Ansprüche 1 bis 11, **dadurch gekennzeichnet, dass** der durch die Strahler-Detektor-Elemente (11, 12, 13, 14) gebildete Strahler-Detektor-Ring (10) eine Polygonform beschreibt, wobei mehrere starr miteinander verbundene Strahler-Detektor-Elemente (12, 13, 14) gegenüber dem restlichen Strahler-Detektor-Ring (10) verschwenkbar sind.

15. Computertomograph nach einem der Ansprüche 1 bis 14, **dadurch gekennzeichnet, dass** sämtliche Strahler-Detektor-Elemente (11, 12, 13, 14) eine einheitliche Form aufweisen.

**Claims**

1. A computer tomograph having a plurality of X-ray tubes (3) which are arranged in a fixed angular position about a geometric center axis (MA) and which are components of emitter-detector elements (11, 12, 13, 14) which together form an emitter-detector ring (10) to be opened by displacement of at least one of the emitter-detector elements (11, 12, 13, 14), wherein each X-ray tube (3) comprises at least one cathode (5, 25) provided for the emission of electrons and an associated anode arrangement (9) with at least one anode (6), wherein, as to a direction of emission of the electrons, each cathode (5, 25) has an alignment angle ($\alpha$) with respect to the geometrical center axis (MA), and wherein a tangential plane (TE) placed on the focal spot (BF⁻, BF, BF⁺) of the anode (6) has a surface normal (FN) which encloses an anode angle ($\beta$) with the center axis (MA), and wherein the X-ray radiation (RS) emanating from the focal spot (BF⁻, BF, BF⁺) is directed at a mean radiation angle ($\gamma$), to be measured with respect to a radial line (RL) extending through the focal spot (BF), to an X-ray detector (4) which is arranged offset with respect to the X-ray tube (3) in an axial direction relative to the geometric center axis (MA), wherein the quotient formed by the sum of the alignment angle ($\alpha$) and the radiation angle ($\gamma$) and by the anode angle ($\beta$) is at least two-ninths and at most two, and wherein each cathode (5, 25) in cooperation with an electrode arrangement (21) of the X-ray tube (3) is configured for generating a focal spot (BF⁻, BF, BF⁺) at one of at least three selectable positions on the anode arrangement (9).

2. The computer tomograph according to claim 1, **characterized in that** the alignment angle ($\alpha$) is greater than zero and at most 30°.

3. The computer tomograph according to claim 1 or 2, **characterized in that** the anode angle ($\beta$) is at least 10° and at most 60°.

4. The computer tomograph according to any one of claims 1 to 3, **characterized in that** the mean radiation angle ($\gamma$) is at least 5° and at most 30°.

5. The computer tomograph according to any one of claims 1 to 4, **characterized in that** the anode (6) is designed as a fixed anode.

6. The computer tomograph according to claim 5, **characterized in that** the anode (6) is designed as an uncooled anode.

7. The computer tomograph according to claim 5, **characterized in that** the anode (6) is designed as a liquid-cooled anode.

8. The computer tomograph according to any one of claims 1 to 7, **characterized in that** the quotient formed from the sum of the alignment angle ($\alpha$) and the radiation angle ($\gamma$) as the numerator and from the anode angle ($\beta$) as the denominator is at least 2/5 and at most 8/5.

9. The computer tomograph according to any one of claims 1 to 8, **characterized in that** the cathode (5) is designed for field emission of electrons and comprises nanorods, in particular carbon nanotubes.

10. The computer tomograph according to claim 9, **characterized in that** each emitter-detector element (11, 12, 13, 14) includes at least one emitter (5) of a first type and at least one emitter (25) of a second type, wherein the different emitter types (5, 25) within an emitter-detector element (11, 12, 13, 14) differ from each other with respect to their materials and/or geometry.

11. The computer tomograph according to claim 9 or 10, **characterized in that** the emitter-detector elements (11, 12, 13, 14) are designed for switching between different X-ray frequencies and/or X-ray doses, wherein each focal spot (BF-, BF, BF⁺) is equally selectable as source of all adjustable X-ray frequencies and X-ray doses.

12. The computer tomograph according to any one of claims 1 to 11, **characterized in that** the emitter-detector ring (10) formed by the emitter-detector elements (11, 12, 13, 14) describes a segmented circular shape, wherein at least one emitter-detector element (11, 12, 13, 14) designed as a segment is displaceable, in particular pivotable, with respect to the other emitter-detector elements (11, 12, 13, 14).

13. The computer tomograph according to claim 12, **characterized in that** each emitter-detector element (11, 12, 13, 14) to be opened is individually pivotable.

14. The computer tomograph according to any one of claims 1 to 11, **characterized in that** the emitter-detector ring (10) formed by the emitter-detector elements (11, 12, 13, 14) describes a polygonal shape, wherein a plurality of rigidly interconnected emitter-detector elements (12, 13, 14) can be pivoted relative to the remaining emitter-detector ring (10).

15. The computer tomograph according to any one of claims 1 to 14, **characterized in that** all the emitter-detector elements (11, 12, 13, 14) have a uniform shape.

**Revendications**

1. Tomographe assisté par ordinateur, pourvu d'une pluralité de tubes à rayons X (3) placés selon une position angulaire déterminée autour d'un axe médian (MA) géométrique, lesquels sont des composants d'éléments émetteurs-détecteurs (11, 12, 13, 14), qui créent ensemble un anneau émetteur-détecteur (10) qui doit s'ouvrir par déplacement d'au moins l'un des éléments émetteurs-détecteurs (11, 12, 13, 14), chaque tube à rayons X (3) comportant au moins une cathode (5, 25) prévue pour l'émission d'électrons et un ensemble d'anodes (9) associé, comprenant au moins une anode (6), en ce qui concerne la direction d'émission des électrons, chaque cathode (5, 25) présentant un angle d'orientation ($\alpha$) par rapport à l'axe médian (MA) géométrique, et un plan tangentiel (TE) appliqué sur le foyer (BF⁻, BF, BF⁺) de l'anode (6) présentant une normale à la surface (FN) qui inclut un angle d'anode ($\beta$) avec l'axe médian (MA) et le rayonnement X (RS) partant du foyer (BF-, BF, BF+) étant orienté sous un angle de rayonnement ($\gamma$) moyen, à mesurer par rapport à une ligne radiale (RL), passant à travers le foyer (BF) vers un détecteur de rayons X (4), qui par rapport au tube à rayons X (3) en direction axiale, rapportée à l'axe médian (MA) géométrique, est placée en décalage, le quotient formé à partir de la somme de l'angle d'orientation ($\alpha$) et de l'angle de rayonnement ($\gamma$), et de l'angle d'anode ($\beta$) étant de deux neuviè-

mes et d'au plus deux et chaque cathode (5, 25) étant conçue pour générer en coopération avec un ensemble d'électrodes (21) du tube à rayons X (3) un foyer (BF⁻, BF, BF⁺) sur une parmi au moins trois positions sélectionnables sur l'ensemble d'anodes (9).

2. Tomographe assisté par ordinateur selon la revendication 1, **caractérisé en ce que** l'angle d'orientation ($\alpha$) est supérieur à zéro et est d'un maximum de 30°.

3. Tomographe assisté par ordinateur selon la revendication 1 ou 2, **caractérisé en ce que** l'angle d'anode ($\beta$) est d'au moins 10° et d'un maximum de 60°.

4. Tomographe assisté par ordinateur selon l'une quelconque des revendications 1 à 3, **caractérisé en ce que** l'angle de rayonnement ($\gamma$) moyen est d'au moins 5° et d'un maximum de 30°.

5. Tomographe assisté par ordinateur selon l'une quelconque des revendications 1 à 4, **caractérisé en ce que** l'anode (6) est conçue sous la forme d'une anode stationnaire.

6. Tomographe assisté par ordinateur selon la revendication 5, **caractérisé en ce que** l'anode (6) est conçue sous la forme d'une anode non refroidie.

7. Tomographe assisté par ordinateur selon la revendication 5, **caractérisé en ce que** l'anode (6) est conçue sous la forme d'une anode refroidie par liquide

8. Tomographe assisté par ordinateur selon l'une quelconque des revendications 1 à 7, **caractérisé en ce que** le quotient, qui est formé à partir de la somme de l'angle d'orientation (a) et de l'angle de rayonnement ($\gamma$) en tant que numérateur et de l'angle d'anode ($\beta$) en tant que dénominateur est d'au moins, 2/5 et d'au plus 8/5.

9. Tomographe assisté par ordinateur selon l'une quelconque des revendications 1 à 8, **caractérisé en ce que** la cathode (5) est conçue pour l'émission par effet de champ d'électrons et comprend des nanotiges, notamment des nanotiges en carbone.

10. Tomographe assisté par ordinateur selon la revendication 9, **caractérisé en ce que** chaque élément émetteur-détecteur (11, 12, 13, 14) comporte au moins un émetteur (5) de premier type et au moins un émetteur (25) de deuxième type, différents types d'émetteurs (5, 25) à l'intérieur d'un élément émetteur-détecteur (11, 12, 13, 14) se différenciant au niveau de leurs matières et / ou de leur géométrie.

11. Tomographe assisté par ordinateur selon la revendication 9 ou 10, **caractérisé en ce que** les éléments émetteurs-détecteurs (11, 12, 13, 14) sont conçus pour commuter entre différentes fréquences de rayons X et / ou doses de rayons X, chaque foyer (BF-, BF, BF⁺) étant sélectionnable aussi bien en tant que source de toutes les fréquences de rayons X et doses de rayons X réglables.

12. Tomographe assisté par ordinateur selon l'une quelconque des revendications 1 à 11, **caractérisé en ce que** l'anneau émetteur-détecteur (10) créé par les éléments émetteurs-détecteurs (11, 12, 13, 14) décrit une forme circulaire segmentée, au moins un élément émetteur-détecteur (11, 12, 13, 14) conçu sous la forme d'un segment étant déplaçable, notamment susceptible de pivoter par rapport aux autres éléments émetteurs-détecteurs (11, 12, 13, 14).

13. Tomographe assisté par ordinateur selon la revendication 12, **caractérisé en ce que** chaque élément émetteur-détecteur (11, 12, 13, 14) qui doit être ouvert est susceptible de pivoter individuellement.

14. Tomographe assisté par ordinateur selon l'une quelconque des revendications 1 à 11, **caractérisé en ce que** l'anneau émetteur-détecteur (10) créé par les éléments émetteurs-détecteurs (11, 12, 13, 14) décrit une forme polygonale, plusieurs éléments émetteurs-détecteurs (12, 13, 14) rigidement assemblés les uns avec les autres étant susceptibles de pivoter par rapport à l'anneau émetteur-détecteur (10) restant.

15. Tomographe assisté par ordinateur selon l'une quelconque des revendications 1 à 14, **caractérisé en ce que** tous les éléments émetteurs-détecteurs (11, 12, 13, 14) présentent une forme homogène.

# Fig. 1

# Fig. 2

# Fig. 3

## Fig. 4

# Fig. 6

# Fig. 7

**Fig. 8**

**Fig. 9**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2018086744 A2
- DE 102007046278 A1
- DE 102010020604 A1
- DE 3426934 A1
- RU 216081 C2
- DE 10237546 B4
- WO 2019057339 A1
- WO 2019042587 A2
- WO 2018086737 A1
- WO 2018141485 A1
- DE 102017008810 A1
- WO 2019057338 A1
- DE 102014215548 A1